# EUROPEAN PATENT APPLICATION

(11) **EP 1 489 093 A1**
(43) Date of publication of application: **22.12.2004**
(21) Application number: 03712764.4
(22) Date of filing: 19.03.2003
(51) Int. Cl.: C07K 14/47, C12N 15/12, C12N 5/10, C12P 21/02, A61K 38/00, A61K 48/00

(54) **PEPTIDES, MEDICINAL COMPOSITIONS CONTAINING THE PEPTIDE AND MEDICINAL COMPOSITIONS FOR TREATING CANCER**

(30) Priority: 19.03.2002 JP 2002075575
(71) Applicant: Oncorex, Inc., Sapporo-shi, Hokkaido 060-0063 (JP)
(72) Inventor: KOBAYASHI, Masanobu Institute For Genetic Medicine, Sapporo-shi, Hokkaido 060-0815 (JP)
(74) Representative: Bassil, Nicholas Charles
(86) International application number: PCT/JP2003/003344
(87) International publication number: WO 2003/078460

(57) **Abstract**

Peptides comprising the amino acid sequence set forth in SEQ ID NO: 1, in which at least one amino acid is deleted from the N-terminal side, are demonstrated herein to inhibit angiogenesis of cancer cells and suppress the proliferation of cancer cells due to that inhibitory effect. Accordingly, the peptides of the present invention described herein can be used to treat various cancers, such as stomach cancer, colorectal cancer, lung cancer, ovarian cancer, liver cancer, and pancreatic cancer.

## Description

### Technical Field

The present invention relates to peptides that are effective in treating cancer, pharmaceutical compositions comprising the same, and the like.

### Background Art

Recently, cancer has become the second cause of human death following heart disease. Cancer is usually treated by surgical operation, radiotherapy, chemotherapy, immunotherapy, hyperthermia, etc. In all these treatments, the removal of viable cells and induction of cell death are important objectives in order to exterminate cancer cells.

As mentioned above, the goal of cancer treatment is extermination of cancer cells. Gene therapy methods for introducing into cancer cells genes that induce cancer cell death or enhance immune response are being developed.

However, regarding gene therapy methods that involve the introduction genes that induce cancer cell death, there is currently no method to introduce genes into all cancer cells. Thus, it is difficult to achieve the intended goal, i.e., the extermination of cancer cells. Furthermore, regarding gene therapy methods involving the introduction of genes that enhance immune response, since the immune response is complicatedly regulated at multiple steps, it has proven to be difficult to enhance the immune response by manipulating one gene alone.

On the other hand, for disorders caused by the depletion of a particular intracellular substance, such as immunodeficiency, gene therapy methods introducing into cells genes that supplement the substance have been shown to be successful. Therefore, such a strategy may also be important in gene therapy for cancer, namely, treating cancer by producing a certain substance.

Adrenomedullin, first discovered in human pheochromocytoma, is a peptide comprising 52 amino acids (Kitamura, K. et al. "Adrenomedullin, a novel hypotensive peptide isolated from human pheochromocytoma", Biochem. Biophys. Res. Commun. 192: 553-560 (1993)). Adrenomedullin is a hypotensive peptide produced from a preprohormone comprising 185 amino acids through successive enzymatic degradations and amidation. Through these enzymatic degradation and amidation steps, adrenomedullin comprising 52 amino acids is produced.

Adrenomedullin is present in many tissues, including normal adrenal/medulla, atrium, ventricle, endothelial cell, lung, brain, kidney, and bone, and is known to have vasodilating activity. Adrenomedullin is also involved in angiogenesis among cells, through its vasodilating activity. The adrenomedullin content has been shown to be higher in various cancer cells as compared to normal cells. Since angiogenesis is necessary for the proliferation of cancer cells, to treat cancer, cancer cell proliferation can be suppressed by inhibiting angiogenesis.

Therefore, a compound capable of inhibiting adrenomedullin-induced angiogenesis, once discovered, may prove to be efficacious in treating various cancers.

Accordingly, an objective of the present invention is to provide novel compounds usable in treating various cancers, such as stomach cancer, colorectal cancer, lung cancer, ovarian cancer, liver cancer, and pancreatic cancer.

### Disclosure of the Invention

Noting that adrenomedullin is expressed at high levels in cancer cells and involved in angiogenesis, the present inventors considered the use of antagonists of adrenomedullin in cancer treatment and searched for novel compounds usable in treating cancer.

As a result of exhaustive studies, the present inventors have discovered that a peptide comprising a partially deleted amino acid sequence of adrenomedullin has cytotoxicity against cancer cells, and.thus is effective in treating cancer.

Accordingly, the present invention provides peptides comprising amino acid sequences wherein at least one amino acid has been deleted from the N-terminal side of the amino acid sequence set forth in SEQ ID NO: 1.

The present invention also provides peptides for treating cancer, comprising amino acid sequences wherein at least one amino acid has been deleted from the N-terminal side of the amino acid sequence set forth in SEQ ID NO: 1.

Furthermore, the present invention provides genes comprising DNA having nucleotide sequences encoding the above-described peptides or peptides for treating cancer.

Moreover, the present invention provides recombinant vectors comprising DNA encoding the above-described peptides or peptides for treating cancer.

In addition, the present invention provides transformants comprising the aforementioned recombinant vectors.

The present invention further provides methods for producing the above-described peptides or peptides for treating cancer, comprising the steps of culturing the transformants, producing and accumulating the peptides or peptides for treating cancer, and collecting the peptides.

The present invention also provides pharmaceutical compositions, particularly pharmaceutical compositions for treating cancer, comprising the above-described peptides or peptides for treating cancer.

### Brief Description of the Drawings

Fig. 1 depicts the expression of adrenomedullin mRNA upon cultivating various cell lines under hypoxic conditions.
Fig. 2 depicts changes in tumor size due to administration of a peptide of the present invention to the tumor.
Fig. 3 is a photograph depicting tumors extirpated from mice.
Fig. 4 depicts the results of weighing tumors extirpated from mice.
Fig. 5 is a series of photographs showing the results of staining tumor tissues with the anti-CD31 antibody.
Fig. 6 is a series of photographs showing the results of staining tumor cells with the anti-PCNA antibody.

### Best Mode for Carrying out the Invention

Hereinafter, the present invention is described in detail.

Peptides of the present invention comprise an amino acid sequence in which at least one amino acid has been deleted from the N-terminal side of a peptide consisting of the amino acid sequence set forth in SEQ ID NO: 1. Specifically, peptides of the present invention comprise amino acid sequences in which 1 to 25, 1 to 20, 1 to 15, 1 to 10, or 1 to 5 amino acids have been deleted from the N-terminal side of a peptide consisting of the amino acid sequence set forth in SEQ ID NO: 1. Herein, the peptide consisting of the amino acid sequence set forth in SEQ ID NO: 1 is adrenomedullin of human origin.

Peptides of the present invention can be obtained by cleaving the peptide consisting of the amino acid sequence set forth in SEQ ID NO: 1 with proteolytic enzymes using methods known in the art.

The nucleotide sequence of human adrenomedullin cDNA has been determined (Kitamura, et al., Biochem. Biophys. Res. Commun. 194, 720 (1993)). Therefore, a desired peptide can be obtained by synthesizing DNA comprising the nucleotide sequence encoding the desired peptide, incorporating the synthesized DNA into a vector, transforming a host with the vector comprising the DNA, and culturing the transformant thus obtained to produce the peptide. Alternatively, peptides of the present invention can be produced by any peptide synthesis method conventionally known in the art.

Peptides of the present invention may also be those in which 1 to 10, preferably 1 to 5, amino acids in the C-terminal side have been deleted. Such peptides, having amino acid deletion in the C-terminal side, can be prepared by the peptide synthesis method or transformant method as described above.

Peptides of the present invention can be used as compositions for treating cancer. Some amino acids may be deleted, substituted, or added, so long as the resulting peptide exerts a therapeutic effect on cancer. In general, peptides are considered substantially identical even when 1 to 5, more preferably 1 to 3, amino acids of the whole amino acid sequence of a peptide are deleted, substituted, or added.

The C-terminus of a peptide of the present invention is usually a carboxyl group (-COOH) or carboxylate (-COO⁻), but may also be an amide (-CONH₂) or ester (-COOR) . Herein, examples of R in esters include C₁₋₆ alkyl groups such as methyl, ethyl, n-propyl, isopropyl, and n-butyl; C₃₋₈ cycloalkyl groups such as cyclopentyl and cyclohexyl; C₆₋₁₂ aryl groups such as phenyl and α-naphthyl; and pivaloyloxymethyl ester, which is widely used as an oral ester.

In addition, when the above-described peptides have a carboxyl group (or carboxylate) at positions other than the C-terminus, such carboxyl groups may be amidated or esterified. For example, esters, such as the aforementioned C-terminal esters, may be used as peptides of the present invention. Furthermore, the present invention also include peptides whose glutamic acid residue at the N-terminus generated by an in vivo cleavage is converted into pyroglutamic acid; peptides in which OH, COOH, NH₂, SH, and such on the side chains of intramolecular amino acids are protected with appropriate protecting groups (*e.g.*, C₁₋₆ acyl groups such as formyl group and acetyl group) ; and conjugated proteins, such as glycoproteins, which are bound with sugar chains.

Salts of the above-described peptides are preferably those of physiologically acceptable acids in particular. Such salts include those with inorganic acids (e.g., hydrochloric acid, phosphoric acid, hydrobromic acid, and sulfuric acid) and with organic acids (e.g., acetic acid, formic acid, propionic acid, fumaric acid, maleic acid, succinic acid, tartaric acid, citric acid, malic acid, oxalic acid, benzoic acid, methanesulfonic acid, and benzenesulfonic acid).

Peptides for treating cancer of the present invention are described below.

Peptides for treating cancer of the present invention are those having the amino acid sequence wherein at least one amino acid has been deleted from the N-terminal side of a peptide consisting of the amino acid sequence set forth in SEQ ID NO: 1. Specifically, the peptides of the present invention have the amino acid sequence where 1 to 25, 1 to 20, 1 to 15, 1 to 10, or 1 to 5 amino acids have been deleted from the N-terminal side of a peptide consisting of the amino acid sequence set forth in SEQ ID NO: 1. Herein, the peptide consisting of the amino acid sequence set forth in SEQ ID NO: 1 is adrenomedullin of human origin.

Peptides for treating cancer of the present invention can be obtained by cleaving the peptide consisting of the amino acid sequence set forth in SEQ ID NO: 1 with a proteolytic enzyme using methods known in the art.

The nucleotide sequence of the human adrenomedullin cDNA has been determined (Kitamura, et al. Biochem. Biophys. Res. Commun. 194, 720 (1993)). Therefore, a desired peptide for treating cancer can be obtained by synthesizing DNA comprising the nucleotide sequence encoding the desired peptide, incorporating this synthesized DNA into a vector, transforming a host with the vector comprising the DNA, and culturing the transformant thus obtained to produce the peptide. The peptide can also be produced using conventional peptide synthesis methods known in the art.

Peptides for treating cancer of the present invention may also be those in which 1 to 10, preferably 1 to 5, amino acids in the C-terminal side have been deleted. Such peptides for treating cancer, having amino acid deletion in the C-terminal side, can be prepared by the peptide synthesis method or the transformant method as described above.

In peptides for treating cancer of the present invention, similarly as in the above-described peptides of the present invention, other amino acids may be deleted, substituted, or added.

Further, the amides, esters, or salts of the peptides for treating cancer are similar to those of the above-described peptides of the present invention.

A specific example of a peptide and peptide for treating cancer of the present invention is the peptide comprising the amino acid sequence set forth in SEQ ID NO: 2.

Peptides and peptides for treating cancer of the present invention can be used in the treatment of various cancers, such as stomach cancer, colorectal cancer, lung cancer, ovarian cancer, liver cancer, and pancreatic cancer. When introduced into cancer cells, the peptides and peptides for treating cancer of the present invention are believed to inhibit angiogenesis of cancer cells and suppress the proliferation of cancer cells through such an inhibitory effect.

Anti-cancer activities of the peptides and peptides for treating cancer of the present invention can be assessed, for example, by administering these peptides to animals, such as mice, in which caner has been generated, and investigating the disappearance of cancer cells.

Next, genes having DNA comprising the nucleotide sequence encoding the peptides or peptides for treating cancer of the present invention (hereinafter also simply referred to as "peptides") are described. As described above, the cDNA of the human adrenomedullin has been sequenced. Therefore, based on the published nucleotide sequence, a desired peptide can be obtained by synthesizing a DNA molecule comprising the nucleotide sequence encoding the desired peptide.

A recombinant vector comprising DNA encoding a peptide of the present invention can be prepared according to conventional methods known in the art, for example, by ligating (inserting) a nucleotide sequence encoding a peptide of the present invention to an appropriate vector. The present invention is not limited to a particular vector, so long as the vector can replicate in a selected host. Examples include plasmid DNA and phage DNA.

A recombinant vector can be prepared by excising a DNA fragment comprising DNA encoding a peptide of the present invention, and ligating it downstream of a promoter within an appropriate expression vector. Suitable vectors for use in the present invention include plasmids derived from *Escherichia coli* (*e.g.*, pBR322, pBR325, pUC18, pUC19, pUC118, or pBluescript); plasmids derived from *Bacillus subtilis* (e.g., pUB110, pTP5, or pC194); plasmids derived from yeast (e.g., pSH19, pSH15, YEp13, or YCp50); bacteriophages, such as λ phage; and animal viruses, such as retrovirus, vaccinia virus, or baculovirus. In the context of the present invention, any promoters may be used so long as they are adapted for use in hosts used to express the gene of interest. For example, preferred promoters are as follows: when the host is *E. coli,* the trp promoter, lac promoter, recA promoter, λPL promoter, 1pp promoter, T7 promoter, T3 promoter, and araBAD promoter; when the host belongs to the genus *Bacillus,* the SPO1 promoter, penP promoter, XYL promoter, HWP promoter, and CWP promoter ; when the host is *Bacillus* subtilis, the SPO1promoter, SPO2 promoter, and penP promoter; and when the host is yeast, the PHO5 promoter, PGK promoter, GAP promoter, and ADH promoter. When animal cells are used as the host, usable promoters include the SRα promoter, SV40 promoter, LTR promoter, CMV promoter, HSV-TK promoter, etc. In addition, when insect cells are used as the host, the polyhedron promoter, OplE2 promoter, and such are preferred.

In addition to the above described promoter, the expression vector may include, if desired, an enhancer, splicing signal, poly (A) addition signal, selection marker, SV40 replication origin (abbreviated to "SV40ori" sometimes hereinafter), and such, which are known in the art. Furthermore, if needed, the protein encodedby a DNAof the present invention can also be expressed as a fusion protein with another protein (e.g., glutathione-S-transferase and protein A). Such fusion proteins can be cleaved using a site-specific protease to divide them into respective proteins.

Examples of suitable host cells include Escherichia bacteria, *Bacillus* bacteria, yeast, insect cells, insects, and animal cells. Specific examples of suitable *Escherichia* bacteria include *Escherichia coli* K12•DH1 (Proc. Natl. Acad. Sci. USA 60, 160 (1968)), JM103 (Nucleic Acids Research 9, 309 (1981)), JA221 (Journal of Molecular Biology 120, 517 (1978)), HB101 (Journal of Molecular Biology 41, 459 (1969) ) , DH5α, and JM109. Examples of suitable *Bacillus* bacteria include *Bacillus subtilis* MI114 (Gene 24, 255 (1983)), 207-21 (Journal of Biochemistry 95, 87 (1984) ), and *Bacillus brevis.* Examplesofsuitable yeasts include *Saccaromyces cerevisiae*AH22, AH22R-, NA87-11A, DKD-5D, 20B-12, *Schizosaccaromyces pombe* NCYC1913, NCYC2036, *Pichia pastoris,* and Hansenula polymorpha. Examples of suitable animal cells include simian COS-7 cells, Vero cells, Chinese hamster ovary cells (hereinafter abbreviated to CHO), Chinese hamster ovary cells deficient in dhfr gene (hereinafter abbreviated to CHO (dhfr⁻) ) , mouse L cells, mouse AtT-20 cells, mouse myeloma cells, rat GH3 cells, human FL cells, and HEK293 cells.

Transformation of the above-described host cells can be carried out according to methods known in the art. Exemplary methods for transforming host cells are described in the following references: Proc. Natl. Acad. Sci. USA 69, 2110 (1972); Gene 17, 107 (1982); Molecular & General Genetics 168, 111 (1979); Methods in Enzymology 194, 182-187 (1991) ; Proc. Natl. Acad. Sci. USA 75, 1929 (1978); Cell Technology (Suppl. 8) New Cell Technology Experimental Protocol 263-267 (1995) (Shujunsha); and Virology 52, 456 (1973).

When a plant is transformed with a gene encoding a peptide of the present invention to obtain a transgenic plant, the gene can be introduced into the plant, for example, by the electroporation method, the *Agrobacterium* method, the particle gun method, or the PEG method. For example, when the electroporation method is used, the gene is introduced into the host in an electroporation device equipped with a pulse controller, under the conditions of 500 to 600 V and 100 µF for 20 msec.

When the *Agrobacterium* method is used, a transgenic plant can be obtained by introducing a plant expression vector construct into an appropriate *Agrobacterium,* such as *Agrobacterium tumefaciens,* and infecting a leaf fragment of a host with such a strain under aseptic conditions according to, for example, the vacuum infiltration method (described in Bechtold et *al.,* C. R. Acad. Sci. Ser. III Sci. Vie, 316, 1194-1199 (1993)).

When the particle gun method is employed, plant bodies, plant organs (*e.g.*, leafs, petals, stems, roots, and seeds), andplant tissues (*e.g.*, epidermis, phloem, parenchyma, xylem, and vascular bundle) are used as is, or sections or protoplasts may be prepared therefrom for use. Samples thus prepared are then treated using a gene transfer apparatus (*e.g.*, BIOLISTIC POS 1000/He, BioRad). The treatment is usually performed under a pressure of about 1000 to 1100 psi and a distance of around 5 to 10 cm; however, treatment conditions may vary depending on plants and samples.

Further, plants usable in transformation may be any of conifers, broad-leaved trees, dicotyledons, monocotyledons, etc.

Methods for introducing recombinant vectors into bacteria such as *E. coli* are not particularly limited, so long as they successfully introduce DNA into the selected bacteria. For example, the calcium ion method (Cohen, S.N. et *al.,* Proc. Natl. Acad. Sci. USA 69, 2110 (1972)) and the electroporation method may be used.

When yeast is used as the host, the methods for introducing recombinant vectors therein are not particularly limited, so long as they allow for successful introduction of DNA into yeast. For example, the electroporation method, the spheroplast method, and the lithium acetate method may be used.

When animal cells are used as the host, the methods for introducing recombinant vectors therein are not particularly limited, so long as they allow for successful introduction of DNA into the animal cells. For example, the electroporation method, the calcium phosphate method, and the lipofection method may be used.

When insect cells are used as the host, methods for introducing recombinant vectors into insect cells are not particularly limited, so long as they allow for successful introduction of DNA into the insect cells. For example, the calcium phosphate method, the lipofection method, and the electroporation method may be used.

Incorporation of a gene into a host can be confirmed, for example, by the PCR method, the Southern hybridization method, and the Northern hybridization method. For example, DNA is prepared from the transformant and DNA-specific primers are designed to perform PCR. PCR is carried out under conditions similar to those used in preparing the above-described plasmids. Then, amplified products are subjected to, for example, agarose gel electrophoresis, polyacrylamide gel electrophoresis, or capillary electrophoresis, and stained with ethidium bromide, SYBR Green solution, etc. The amplified product detected as a single band indicates successful transformation. Amplified products may also be detected by performing PCR using primers labeled with fluorescent dye and such in advance. Furthermore, amplified products can be identified through fluorescence or enzymatic reaction after fixing them on a solid phase, such as a microplate.

A peptide of the present invention can be prepared by culturing an aforementioned transformant, producing and accumulating the peptide, and collecting the peptide. In the context of the present invention, the peptide may be accumulated through the culture not only in culture supernatants but also in any of cultured cells, cultured bacterial cells, or the lysate of cells or bacteria. In the present invention, methods for culturing transformants are not particularly limited, and any method conventionally used in culturing hosts may be used.

For example, when the host is a microorganism, such as *E. coli* or yeast, the culture mediummay be either a natural medium or a synthetic medium, so long as it contains the carbon source, nitrogen source, minerals, and such, to be metabolized by the microorganism, and is capable of efficiently culturing the transformant. Examples of carbon sources include carbohydrates, such as glucose, fructose, sucrose, and starch; organic acids, such as acetic acid and propionic acid; and alcohols, such as ethanol and propanol. Examples of nitrogen sources include ammonia; ammonium salts of inorganic or organic acids, such as ammonium chloride, ammonium sulfate, ammonium acetate, and ammonium phosphate; other nitrogen-containing compounds; and peptone, meat extracts, and corn steep liquor. Examples of mineral sources include potassium dihydrogenphosphate, dipotassium hydrogenphosphate, magnesium phosphate, magnesium sulfate, sodium chloride, ferrous sulfate, manganese sulfate, copper sulfate, and calcium carbonate. The culture is usually performed under aerobic conditions by the shake culture, aerated spinner culture, etc. When the host is *E. coli,* the culture is carried out, at about 15 to 43°C for about 12 to 48 h. When the host is a *Bacillus bacterium,* the culture is carried out at about 30 to 40°C for about 12 to 100 h. When the host is yeast, the culture is performed at about 20 to 35°C for about 24 to 100 h. If necessary, aeration and stirring may be applied to the culture. When pH adjustment is required, it is typically performed using inorganic or organic acid, alkaline solution, etc.

When using an inducible promoter as the promoter in the recombinant expression vector, the culture medium for the transformants comprising the expression vectormay include the addition of an inducer if necessary. For example, when the expression vector of interest includes the T7 promoter, culture may be performed with the addition of IPTG and such to the medium. Furthermore, when the expression vector of interest includes the trp promoter, a promoter inducible with indoleacetic acid (IAA), IAA and such may be added to the medium.

When culturing transformants obtained using animal cells as the host, suitable media include the generally used RPMI1640 medium, DMEM medium, or these media supplemented with fetal bovine serum and such. The culture is usually performed in the presence of about 5% carbon dioxide at about 37°C for 1 to 30 days.

When a peptide of the present invention is produced through cell culture, a crude extract of the protein may be obtained, for example, by a method comprising: collecting the cells by methods known in the art; suspending them in an appropriate buffer solution; crushing the cells by, for example, sonication, lysozyme, and/or freeze-thawing; and then centrifuging and/or filtering. The buffer solution may contain protein denaturants, such as urea and guanidine hydrochloride, and surfactants, such as Triton X-100®. When the peptide is secreted into the culture medium, the supernatant is collected after the culture and separated from the cells using methods known in the art. The proteins contained in the supernatant or extract thus obtained can be purified by appropriate combinations of separation/purification methods known in the art. That is, the protein of interest can be purified using, for example, ammonium sulfate precipitation, gel chromatography, ion exchange chromatography, and affinity. chromatography, either alone or in appropriate combinations.

A peptide of the present invention thus obtained can be converted into a salt by methods known in the art or methods similar to those already known. Similarly, when the peptide is obtained as a salt, it can be converted to the free form or another salt form using methods known in the art or methods similar to those already known. Furthermore, proteins produced by transformants can optionally be fragmented, before or after purification, by treatment with appropriate protein modifying enzymes, such as trypsin and chymotrypsin. In addition, proteins can optionally be modified by treatment with protein modifying enzymes such as kinases. The presence in a sample of a protein of the present invention or a salt thereof can be determined using various binding assays, enzyme immunoassays with specific antibodies, etc.

Pharmaceutical compositions or pharmaceutical compositions for treating cancer of the present invention are explained below. Pharmaceutical compositions or pharmaceutical compositions for treating cancer of the present invention contain a peptide or peptide for treating cancer of the present invention. The pharmaceutical compositions or pharmaceutical compositions for treating cancer of the present invention contain pharmaceutically acceptable carriers in addition to a peptide or peptide for treating cancer of the present invention. The content of the peptide of the present invention is defined as an amount capable of suppressing cancer cell growth, reducing cancer cells, or exhibiting therapeutic effect on treated patients, when administered to a patient in need thereof. Dosages to be administered to a patient are generally determined based on the body surface area, body weight, symptom, etc. of the patient. The mutual relationship of dosages between animals and humans is known in the art, and the body surface area of a patient can be determined from the height and body weight of patients.

Suitable dosages of peptides of the present invention may range from about 0.1 mg/kg to about 0.5 mg/kg . Dosages are preferably altered according to the administration method and amount of excipient(s); dosages are further altered when other treatment methods, such as other anticancer drugs and radiotherapy, are combined with the peptides.

Pharmaceutical compositions or pharmaceutical compositions for treating cancer of the present invention can be administered parenterally, for example, subcutaneously, intraperitoneally, intramuscularly, or intravenously. An embodiment of a pharmaceutical preparation for parenteral administration may include an aqueous solution containing about 5% glucose or other pharmaceutically usable excipients and activators known in the art for use in an isotonic salt solution. Solubilizers, such as cyclodextrin and other solubilizers known in the art, are examples of excipients that may be added.

Pharmaceutical compositions or pharmaceutical compositions for treating cancer of the present invention can be incorporated into a pharmaceutical preparation for administration by other methods known in the art using conventional techniques. For example, the pharmaceutical compositions or pharmaceutical compositions for treating cancer can be incorporated into pharmaceutical preparations for oral administration, such as capsules, gels, or tablets. Capsules are composed of pharmaceutically acceptable materials known in the art, such as gelatin or cellulose derivatives. Tablets can be obtained by compressing mixtures of peptides of the present invention and solid carriers as well as lubricants, using methods known in the art. Examples of suitable solid carriers include starch and sugar bentonite. The peptides of the present invention can be administered, for example, in the form of hard shell tablets containing lactose or mannitol as binders, known filling materials, and/or tablet-forming reagents, or in the form of capsules.

Pharmaceutical compositions or pharmaceutical compositions for treating cancer comprising peptides or peptides for treating cancer of the present invention can be used to treat various cancers, such as stomach cancer, colorectal cancer, lung cancer, ovarian cancer, liver cancer, and pancreatic cancer. When introduced into cancer cells, the peptides or peptides for treating cancer contained in pharmaceutical compositions or pharmaceutical compositions for treating cancer of the present invention are believed to inhibit angiogenesis of cancer cells, and suppress the proliferation of cancer cells due to that inhibitory effect.

The anti-cancer activity of a pharmaceutical composition or pharmaceutical composition for treating cancer of the present invention can be assessed by generating cancer in animals such as mice, then administering to the animals the pharmaceutical composition or pharmaceutical composition for treating cancer and examining the disappearance of cancer.

In an alternate embodiment, pharmaceutical compositions or pharmaceutical compositions for treating cancer of the present invention may contain genes comprising DNA encoding peptides or peptides for treating cancer of the present invention.

Pharmaceutical compositions or pharmaceutical compositions for treating cancer of the present invention can be administered in a dosage form using either non-viral vector or viral vectors.

When using a non-viral vector, DNA encoding a peptide or peptide for treating cancer of the present invention can be introduced into cells or tissues using a recombinant expression vector prepared by incorporating the DNA into a commonly used gene expression vector employing techniques as described below. Examples of methods for introducing genes into cells include the calcium phosphate co-precipitation method and the DNA direct injection method using glass micropipette.

Additional examples of methods for introducing genes into tissues include gene transfer methods using internal type liposome, electrostatic type liposomes, HVJ-liposomes, and improved HVJ-liposomes (HVJ-AVE liposome); gene transfer methods mediated by receptors; methods for transferring DNA molecules together with carriers (metal particles) into cells using a particle gun; methods for directly transferring naked-DNA; and transfer methods mediated by positively charged polymers. Herein, suitable expression vectors include pCAGGS (Gene 108, 193-200(1991)), pBK-CMV, pcDNA3.1, and pZeoSV (Invitrogen, Stratagene).

Viral vectors suitable for administration are exemplified by recombinant adenoviruses and retroviruses. Specifically, genes can be introduced into cells using recombinant expression vectors comprising DNA encoding a peptide of the present invention incorporated into DNA viruses or RNA viruses, such as retrovirus, adenovirus, adeno-associated virus, herpes virus, vaccinia virus, poxvirus, poliovirus, Sindbis virus, Sendai virus, SV40, and human immunodeficiency virus (HIV), which have been made a virulent, to infect cells with these recombinant viruses.

Among the aforementioned viral vectors, the infection efficiency of adenovirus is known to be extremely high as compared to other viral vectors. From this point of view, the adenoviral vector system is preferably used.

Pharmaceutical compositions or pharmaceutical compositions for treating cancer of the present invention can be introduced into patients by the in vivo method for directly introducing pharmaceutical compositions into the body of a patient, or by the ex vivo method for introducing pharmaceutical compositions into a certain type of cells extracted from a patient and then returning the cells back into the body of the patient.

When pharmaceutical compositions of the present invention are administered by the in vivo method, they can be administered through an appropriate route according to the target to be treated, i.e., cells, tissues, and target organs. For example, they may be administered intravenously, intra-arterially, subcutaneously, intracutaneously, intramuscularly, or directly into tissues where the target cancer lesions are localized.

Various pharmaceutical preparation forms (*e.g.*, liquid) are suitable for the above-described administration forms and can be routinely adopted. For example, in the case of an inj ection comprising DNA as an effective ingredient, the inj ection can be prepared according to conventional methods by dissolving DNA in an appropriate solvent (*e.g.*, a buffer solution such as PBS, physiological saline, and sterilized water) , sterilizing the solution by filtration with a filter and such, if necessary, and then filling the solution into aseptic containers. Commonly used carriers and such may be added to the injection if necessary. Furthermore, liposomes, such as the HVJ-liposome, can be in the form of a liposome preparation, such as a suspension, frozen form, or centrifugation-concentrated frozen form.

Further, in order to facilitate the localization of the gene into the vicinity of the affected region, a sustained release preparation *(e. g.* mini-pellet) can be prepared to be embedded near the affected region. It is also possible to continuously and slowly administer the gene to the affected area using an osmotic pump or the like.

Selective introduction into cancer cells can be achieved by targeting a cancer antigen specifically expressed on the surface of the cancer cell or a cancer antigen expressed particularly at higher levels in the cancer cell than normal cells (*e. g.* , transferrin receptor and EGF receptor).

For example, pharmaceutical compositions or pharmaceutical compositions for treating cancer of the present invention can be specifically introduced into cancer cells using immuno-liposomes obtained by enclosing a pharmaceutical composition comprising DNA encoding a peptide of the present invention in liposomes coupled with a monoclonal antibody against a specific surface antigen of cancer cells.

Contents of DNA in the pharmaceutical preparations can be appropriately adjusted according to the disease being treated, the age and body weight of the patient, etc.

Pharmaceutical compositions or pharmaceutical compositions for treating cancer containing genes comprising DNA encoding peptides or peptides for treating cancer of the present invention can be used to treat various cancers, such as stomach cancer, colorectal cancer, lung cancer, ovarian cancer, liver cancer, and pancreatic cancer. Following introduction into cells, the pharmaceutical compositions or pharmaceutical compositions for treating cancer of the present invention are believed to produce a peptide of the present invention that inhibits angiogenesis of cancer cells and suppresses the proliferation of cancer cells due to that inhibitory effect.

Peptides or peptides for treating cancer of the present invention can be used to treat cancer as described above. Animals to be treated with the peptides are not particularly limited, and include humans and other mammals, such as rats, monkeys, dogs, cats, mice, guinea pigs, hamsters, rabbits, and wild rabbits.

The anti-cancer activity of a pharmaceutical composition or pharmaceutical composition for treating cancer of the present invention can be assessed, for example, by generating cancer in animals, such as mice, administering the pharmaceutical composition or pharmaceutical'composition for treating cancer, and examining changes in the size of cancer cells.

Hereinafter, the present invention will be explained in more detail with reference to the Examples, but it should not be construed as being limited thereto.

### Example 1

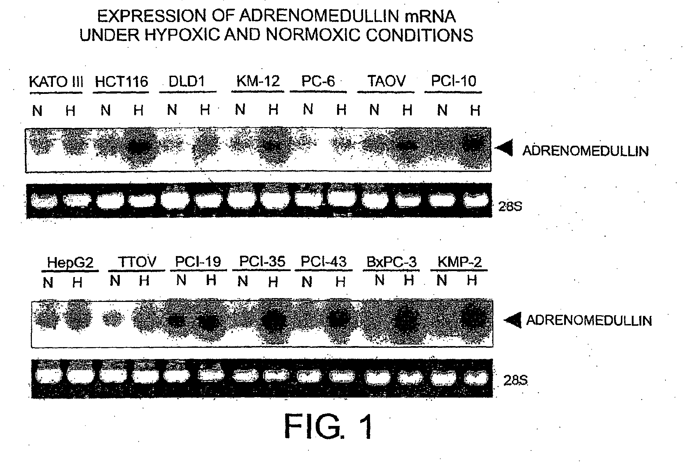

Various cancer cell lines were cultured under hypoxic conditions to examine the expression of adrenomedullin mRNA by the Northern blot method. The culture of cancer cell lines under hypoxic conditions was carried out in 1% O₂ concentration for 12 h using a hypoxic culture chamber (Wakenyaku Industry) . Culture in 20% O₂ concentration was also performed as a control. After the culture, RNA was extracted from various cancer cell lines using the TRIZOL reagent (LIFE TECHNOLOGIES). RNA (20 µg) was electrophoresed on formaldehyde-agarose gel, and then hybridized with an adrenomedullin-specific probe. Results are shown in Fig. 1.

Cancer cell lines used herein were as follows:
- KATO III: Stomach cancer cell line
- HCT116: Colorectal cancer cell line
- DLD1: Colorectal cancer cell line
- KM-12: Colorectal cancer cell line
- PC-6: Lung cancer cell line
- TAOV: Ovarian cancer cell line
- PCI-10: Pancreatic cancer cell line
- HepG2: Liver cancer cell line
- TTOV: Ovarian cancer cell line
- PCI-19: Pancreatic cancer cell line
- PCI-35: Pancreatic cancer cell line
- PCI-43: Pancreatic cancer cell line
- BxPC-3: Pancreatic cancer cell line
- KMP-2: Pancreatic cancer cell line

Fig. 1 depicts the results of the Northern blot analysis of RNA extracted from various cancer cell lines, showing the 28S portion that reacted with the adrenomedullin-specific probe. As shown in Fig. 1, in cancer cells cultured under low oxygen (1% oxygen) concentrations (indicated with H in Fig. 1), adrenomedullin mRNA increases compared to that in cancer cells cultured under normal oxygen (20% oxygen) concentration (indicated with N in Fig. 1). This tendency was, in particular, conspicuous in pancreatic cancer cells.

### Example 2

On the dorsal side of a CB171cr-scid Jl mouse (CLEA JAPAN Inc.) , 10⁷ PCI-43 cells were subcutaneously transplanted. It was confirmed that PCI-43 cells proliferated to form a tumor, and that the tumor diameter exceeded 5 mm after 7 days. After that confirmation, 50 µg each of adrenomedullin and a peptide of the present invention (dissolved in 0.1 ml of physiological saline) was injected into the tumor once a day starting on the 7^{th} day until the 16^{th} day. A peptide consisting of SEQ ID NO: 2 was used as the peptide of the present invention (hereinafter also referred to as "adrenomedullin antagonist"). This peptide was purchased from Wako Pure Chemical Industries, Ltd.

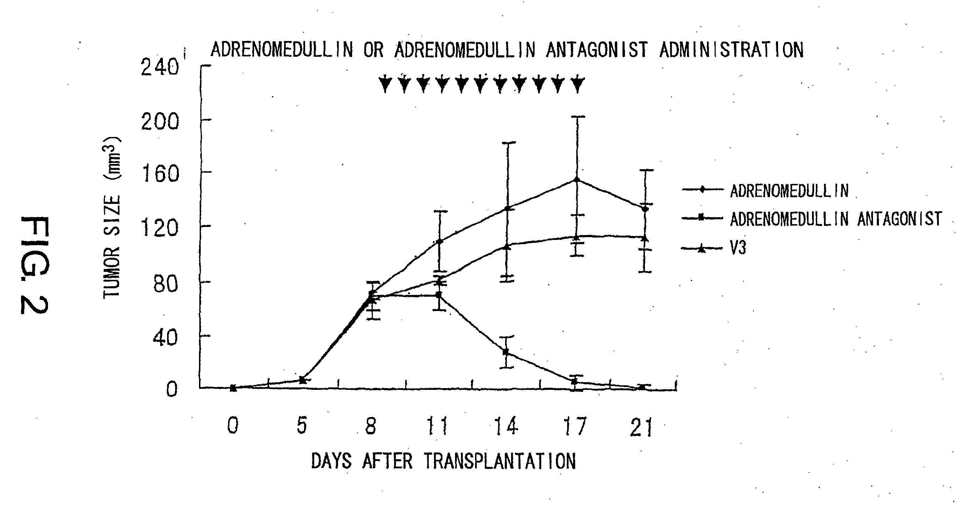

The tumor diameter was megascopically measured every three days. Results are shown in Fig. 2, a graphical representation of tumor sizes when adrenomedullin, adrenomedullin antagonist, and physiological saline (V3), respectively, were administered. The horizontal axis represents the number of days after transplanting PCI-43 cells, and the vertical axis the size of the tumor in volume (mm³) . Arrows at the upper part of the graph indicate timings of administration of adrenomedullin, adrenomedullin antagonist, and physiological saline. Five CB171cr-scid Jl mice were used in each group.

As clearly shown in Fig. 2, in the group in which the adrenomedullin antagonist was administered from the 7^{th} day to 16^{th} day, the tumor size became smaller and on the 21^{st} day became almost megascopically invisible. Conversely, in both the adrenomedullin- and physiological saline-administered groups, the tumor size hardly changed.

### Example 3

On the dorsal side of CB171cr-scid Jl mice (CLEA JAPAN Inc.), 10⁷ PCI-43 cells were subcutaneously transplanted. It was confirmed that PCI-43 cells proliferated to form a tumor, and that the tumor diameter exceeded 5 mm after 7 days. After that confirmation, 50 µg each of adrenomedullin and adrenomedullin antagonist (dissolved in 0.1 ml of physiological saline) was injected into the tumor once every three days from the 7^{th} day, that is, on the 7^{th}, 10^{th}, 13^{th}, and 16^{th} days. Adrenomedullin and adrenomedullin antagonist used herein were the same as those used in Example 1.

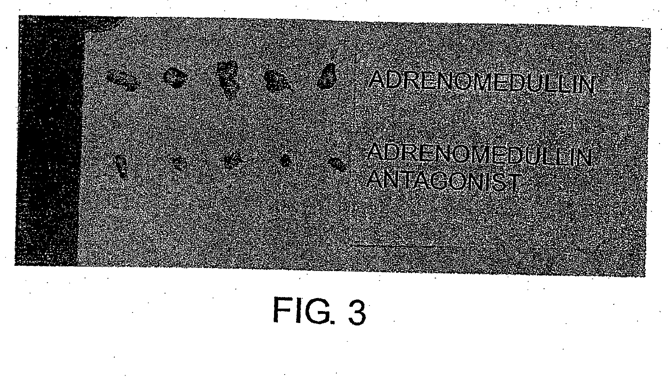

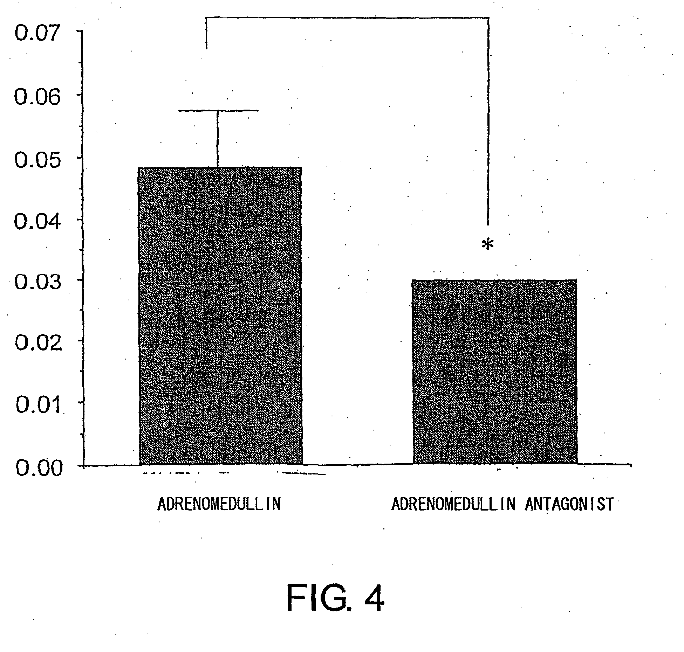

Mice were sacrificed on the 21^{st} day, and tumors were extirpated and weighed. A photograph of the extirpated tumors is shown in Fig. 3, and the results of tumor weighing is shown in Fig. 4.

As clearly seen from Fig. 3, the size of the tumor became smaller in the adrenomedullin antagonist-administered group compared to the adrenomedullin-administered group. Furthermore, as clearly shown in Fig. 4, the tumor weight was about 0.05 g in the adrenomedullin-administered group while that of the adrenomedullin antagonist-administered group was about 0.03 g, decreasing to about half of that of the adrenomedullin-administered group.

### Example 4

The effect of a peptide of the present invention on angiogenesis was examined. Experiments were carried out with the tumor tissues of mice used in Example 3. Mouse tumor tissues were extirpated, and CD31 antigen, which is the cell surface marker specific for vascular endothelial cells, was stained with anti-CD31 antibody. Staining was performed as described below. The experiments were carried out using five mice.

Tumor tissues were frozen in liquid nitrogen to prepare frozen sections. After the frozen sections were pre-treated in albumin solution for 30 min, endogenous peroxidase activity was suppressed with hydrogen peroxide, and then, the sections were treated with anti-CD31 antibody for 1 h at room temperature. After washing, the sections were treated with a secondary antibody for 1 h, and coloured using ECL (Amersham).

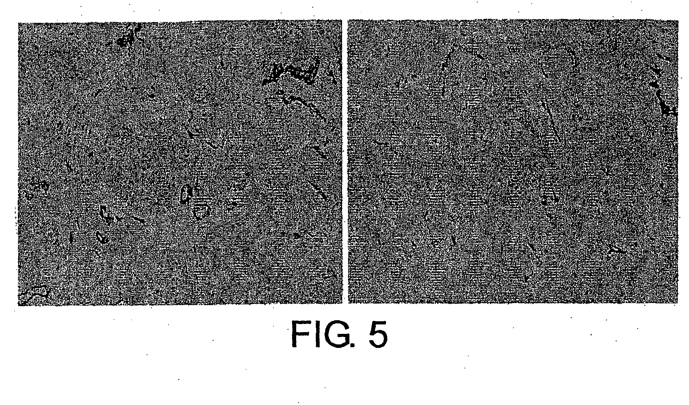

Results of staining are shown in Fig. 5. The staining results for the tumor tissue from an adrenomedullin-administered mouse are shown on the left side of Fig. 5, while those from an adrenomedullin antagonist-administered mouse is shown on the right side. In Fig. 5, the area stained brown represents the newly generated blood vessels. As clearly seen from Fig. 5, in the adrenomedullin-administered group, thick blood vessels were newly generated, while in the adrenomedullin antagonist-administered group, newly generated blood vessels were thin, demonstrating inhibition of angiogenesis.

Next, 100 newly generated blood vessels of the vascular endothelial cells used in Example 4 were arbitrarily selected to measure their respective diameters. In Table 1, results of the number of the newly generated blood vessels classified by their diameters into three groups (less than 2 µm, 2 µm to less than 8 µm, and 8 µm or more) are shown together with the average of blood vessel diameters and standard deviations. In Table 1, AM stands for adrenomedullin, and AMA adrenomedullin antagonist.

**Table 1**

| | Number of blood vessels | | | Average ± standard deviation |
|---|---|---|---|---|
| | less than 2 µm | 2 µm to less than 8 µm | 8 µm or more | |
| AM | 15 | 54 | 31 | 6.454±5.313 |
| AMA | 55 | 44 | 1 | 2.276±1.120 |

As clearly shown in Table 1, in adrenomedullin antagonist-administered mice, the diameters of blood vessels became smaller, with almost no blood vessel having a particularly large diameter, further demonstrating inhibition of angiogenesis. In addition, in the adrenomedullin antagonist-administered mice, blood vessel diameters were one half of those of the adrenomedullin-administered mice.

### Example 5

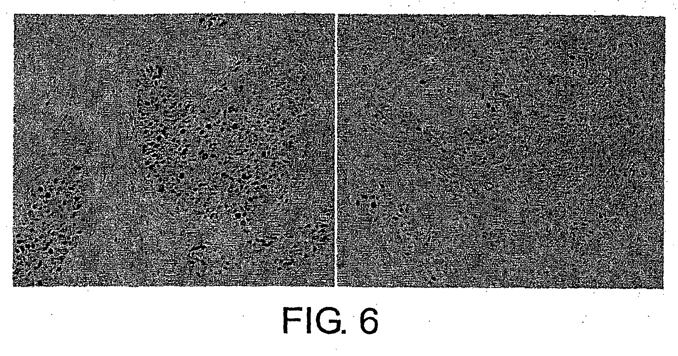

The effect of a peptide of the present invention on cell proliferation was examined. The proliferating cell nuclear antigen (PCNA) of tumor cells from the mice used in Example 2 was stained with anti-PCNA antibody. The staining method was the same as in Example 4. Five mice were used in the experiment. Results are shown in Fig. 6. The staining results for tumor cells of an adrenomedullin-administered mouse are shown on the left side in Fig. 6, while those of an adrenomedullin antagonist-administered mouse are shown on the right side.

As clearly seen from Fig. 6, many cells reacting with the anti-PCNA antibody were present in the adrenomedullin-administered group, while few cells reacted with the anti-PCNA antibody in the adrenomedullin antagonist-administered group. PCNA-labelling indexes were 25.8 ± 3.9 and 6.3 ± 5.2 in the adrenomedullin- and adrenomedullin antagonist-administered groups, respectively. Thus, proliferating cells decreased by about one quarter in the adrenomedullin antagonist-administered mice as compared to the adrenomedullin-administered mice.

As described above in detail, peptides of the present invention inhibit angiogenesis of cancer cells and suppress proliferation of the cells due to their inhibitory effect. Accordingly, peptides of the present invention find utility in treating various cancers, such as stomach cancer, colorectal cancer, lung cancer, ovarian cancer, liver cancer, and pancreatic cancer.

## Claims

1. A peptide comprising the amino acid sequence set forth in SEQ ID NO: 1, in which at least one amino acid is deleted from the N-terminal side.

2. A peptide comprising the amino acid sequence set forth in SEQ ID NO: 1 in which 1 to 25 amino acids are deleted from the N-terminal side.

3. A peptide comprising the amino acid sequence set forth in SEQ ID NO: 1 in which 1 to 20 amino acids are deleted from the N-terminal side.

4. A peptide comprising the amino acid sequence set forth in SEQ ID NO: 1 in which 1 to 15 amino acids are deleted from the N-terminal side.

5. A peptide comprising the amino acid sequence set forth in SEQ ID NO: 1 in which 1 to 10 amino acids are deleted from the N-terminal side.

6. A peptide comprising the amino acid sequence set forth in SEQ ID NO: 1 in which 1 to 5 amino acids are deleted from the N-terminal side.

7. The peptide according to any one of claims 1 to 6, wherein 1 to 10 amino acids are deleted from the C-terminal side.

8. The peptide according to any one of claims 1 to 6, wherein 1 to 5 amino acids are deleted from the C-terminal side.

9. The peptide according to any one of claims 1 to 8, wherein amino acids thereof are partially deleted, substituted, or added.

10. An amide or ester or salt of the peptide according to any one of claims 1 to 9.

11. A peptide for treating cancer comprising the amino acid sequence set forth in SEQ ID NO: 1 in which at least one amino acid is deleted from the N-terminal side.

12. A peptide for treating cancer comprising the amino acid sequence set forth in SEQ ID NO: 1 in which 1 to 25 amino acids are deleted from the N-terminal side.

13. A peptide for treating cancer comprising the amino acid sequence set forth in SEQ ID NO: 1 in which 1 to 20 amino acids are deleted. from the N-terminal side.

14. A peptide for treating cancer comprising the amino acid sequence set forth in SEQ ID NO: 1 in which 1 to 15 amino acids are deleted from the N-terminal side.

15. A peptide for treating cancer comprising the amino acid sequence set forth in SEQ ID NO: 1 in which 1 to 10 amino acids are deleted from the N-terminal side.

16. A peptide for treating cancer comprising the amino acid sequence set forth in SEQ ID NO: 1 in which 1 to 5 amino acids are deleted from the N-terminal side.

17. The peptide for treating cancer according to anyone of claims 1 to 16, wherein 1 to 10 amino acids are deleted from the C-terminal side.

18. The peptide for treating cancer according to any one of claims 1 to 16, wherein 1 to 5 amino acids are deleted from the C-terminal side.

19. The peptide for treating cancer according to any one of claims 1 to 8, wherein amino acids thereof are partially deleted, substituted, or added.

20. An amide or ester or salt of the peptide for treating cancer according to any one of claims 11 to 19.

21. The peptide for treating cancer according to any one of claims 11 to 19, wherein said cancer is stomach cancer, colorectal cancer, lung cancer, ovarian cancer, liver cancer, or pancreatic cancer.

22. The amide or ester or salt of the peptide for treating cancer according to claim 20, wherein said cancer is stomach cancer, colorectal cancer, lung cancer, ovarian cancer, liver cancer, or pancreatic cancer.

23. A gene comprising a DNA having the nucleotide sequence encoding the peptide according to any one of claims 1 to 9 or the peptide for treating cancer according to any one of claims 11 to 19.

24. A recombinant vector comprising a DNA encoding the peptide according to any one of claims 1 to 9 or the peptide for treating cancer according to any one of claims 11 to 19.

25. A transformant comprising the recombinant vector according to claim 24.

26. A method for producing the peptide according to any one of claims 1 to 9 or the peptide for treating cancer according to any one of claims 11 to 19, wherein said method comprises the steps of: culturing the transformant according to claim 25; producing and accumulating the peptide according to any one of claims 1 to 9 or the peptide for treating cancer according to any one of claims 11 to 19; and collecting said peptide.

27. A pharmaceutical composition comprising the peptide according to any one of claims 1 to 9, or the amide or ester or salt of the peptide according to claim 10.

28. A pharmaceutical composition for treating cancer comprising the peptide according to any one of claims 1 to 9, or the amide or ester or salt of the peptide according to claim 10.

29. The pharmaceutical composition for treating cancer according to claim 28, wherein said cancer is stomach cancer, colorectal cancer, lung cancer, ovarian cancer, liver cancer, or pancreatic cancer.

30. A pharmaceutical composition containing a gene comprising a DNA encoding the peptide according to any one of claims 1 to 9 or the peptide for treating cancer according to any one of claims 11 to 19.

31. A pharmaceutical composition for treating cancer containing a gene comprising a DNA encoding the peptide according to any one of claims 1 to 9 or the peptide for treating cancer according to any one of claims 11 to 19.


SEQUENCE LISTING

<110> HOkkaido Technology Licensing Office Co. Ltd.

<120> PEPTIDES AND PHARMACEUTICAL COMPOSITIONS COMPRISING SAID PEPTIDES FOR
TREATING CANCER

<130> PCTF149

<140>
<141>

<150> JP 2002-075575
<151> 2002-03-19

<160> 2

<170> PatentIn Ver. 2.1

<210> 1
<211> 52
<212> PRT
<213> Homo sapiens

<400> 1
Tyr Arg Gln Ser Met Asn Asn Phe Gln Gly Leu Arg Ser Phe Gly Cys

1               5                   10                  15

Arg Phe Gly Thr Cys Thr Val Gln Lys Leu Ala His Gln Ile Tyr Gln

            20                  25                  30

Phe Thr Asp Lys Asp Lys Asp Asn Val Ala Pro Arg Ser Lys Ile Ser

        35                  40                  45

Pro Gln Gly Tyr

    50

<210> 2

<211> 31

<212> PRT

<213> Homo sapiens

<400> 2

Thr Val Gln Lys Leu Ala His Gln Ile Tyr Gln Phe Thr Asp Lys Asp

1               5                   10                  15

Lys Asp Asn Val Ala Pro Arg Ser Lys Ile Ser Pro Gln Gly Tyr

            20                  25                  30